# EUROPEAN PATENT SPECIFICATION

(11) **EP 2 296 616 B1**
(45) Date of publication and mention of the grant of the patent: **24.12.2014**
(21) Application number: 09779892.0
(22) Date of filing: 23.06.2009
(51) Int. Cl.: A61K 8/44, A61Q 19/10, C11D 9/30, C11D 9/34

(54) **A PERSONAL WASH COMPOSITION**
KÖRPERWASCHZUSAMMENSETZUNG
COMPOSITION D'HYGIÈNE PERSONNELLE

(30) Priority: 10.07.2008 IN MU14402008
(43) Date of publication of application: 23.03.2011
(73) Proprietor: Unilever N.V., 3013 AL Rotterdam (NL); Unilever PLC, London Greater London EC4Y 0DY (GB)
(72) Inventor: HOPTROFF, Michael John, Wirral Merseyside CH63 3JW (GB); LEOPOLDINO, Sérgio Roberto, CEP 13271-450 Sao Paulo (BR); MANTHENA, Vamsi Krishna, Mumbai 400 099 (IN); PEDRO, André Messias Krell, CEP 13271-450 Sao Paulo (BR); PLUMMER, Christopher, Ellesmere Port Cheshire CH66 4LP (GB); RAGHAVACHARI, Rajan, Mumbai 400 099 (IN); STEVENS, Deborah, Wirral Merseyside CH63 3JW (GB); TRIVELIN, Luciano Augusto, CEP 13271-450 Sao Paulo (BR)
(74) Representative: Fijnvandraat, Arnoldus
(86) International application number: PCT/EP2009/057813
(87) International publication number: WO 2010/003819

(56) References cited:
- EP-A- 0 696 807
- EP-A- 1 767 612
- WO-A-2006/094586
- WO-A-2008/070015
- DE-A1- 2 020 967
- GB-A- 1 077 743
- US-A- 3 415 752
- US-A- 3 926 828
- US-A- 5 262 079
- US-A1- 2002 031 537

## Description

The present invention relates to personal wash compositions.

The invention has been developed primarily for use in personal wash application and will be described hereinafter with reference to this application. However, it will be appreciated that the invention is not limited to this particular field of use.

Any discussion of the prior art throughout the specification should in no way be considered as an admission that such prior art is widely known or forms part of the common general knowledge in the field.

Personal wash compositions are available in various forms such as bars, liquid soaps, creams and gels. Commercial soap compositions often have one or more "soaps", which means "salts of carboxylic fatty acids". The counter-ions of the salts are generally sodium, potassium, ammonium or alkanolammonium ions, but other suitable ions known in the art are also used, though their use is relatively uncommon. Compositions based on soap bars generally contain from 40% to about 76% total fatty matter, more popularly known in the art in its abbreviated form "TFM". According to Indian legislation, soaps having TFM in the range of 60% to 76% are called "Toilet soaps" and are classified into grades I, II and III depending on the TFM content. On the other hand, soap bars with TFM in the range of 40% to 60% are commonly known in the art as "Bathing bars". The analytical methods for determining the TFM level of soaps are well known in the art. Structuring agents and fillers are generally included in such compositions.

Several bacteria prefer to live and multiply on the human skin. Some of these bacteria are known to cause body odour, pimples and acne. There is some evidence in the prior art to suggest that some of these conditions aggravate in a hot and humid climate. It is known that soaps, i.e. salts of fatty acids, per-se, have antimicrobial properties.

The increasing demand for vegetable oils, such as palm oil, (one of the main sources of oils and fatty acids used by soap manufacturers), and consequent soaring prices has led to severe constraints on the sustainability of the soaps and detergents Industry, as it is becoming increasingly difficult to provide high TFM soaps at a competitive cost, while still making reasonable profits.

As a result, the trend is towards lower TFM soaps, being a cost-effective measure. However, lowering of TFM has a direct and negative effect on the antimicrobial or antibacterial property of the soap compositions. In order to restore the antimicrobial activity, actives such as Triclosan (i.e. 2,4,4'-trichloro-2'-hydroxy-diphenylether; TCN), Triclocarbanilide (TCC), Benzalkonium chloride or other widely recognised antimicrobial agents are generally added to personal wash compositions. However, the long-term, continued use of both TCN and TCC has come under the scanner of environmentalists and consumer bodies due to concerns over bio-degradation. It is therefore desirable to stop using these agents, or at least minimise their use.

Soap bars with relatively lower TFM have been described in prior art.

A low TFM soap bar has been described in WO2006/094586 (HINDUSTAN LEVER LTD). The composition includes 15% to 30% TFM, 25% to 70% inorganic particulates including talc and calcium carbonate, 0.5% to 10% aluminosilicate; and 3% to 20% water. There is no reference to the antibacterial activity of the resultant compositions.

GB2319181 (RECKITT & COLMANN PRODUCTS LTD, 1999) states that the activity of many antimicrobial agents may be improved by the use of chelating agents, and such agents are often used in antiseptic soaps. It is said that chelating agents are often used as stabilizing agents in the soap base but are frequently added again during manufacture of the finished soap, in the belief that they will increase antimicrobial activity. The patent discloses antiseptic soap bar which includes 0.35 to 0.75 wt% of chlorinated methyl substituted phenol (an antimicrobial), 0.15 to 0.25 wt% Triclosan (or a salt thereof); and less than 0.075 wt% EDTA as the chelating agent. It is said that there are a number of drawbacks associated with the use of chelating agents, notably their cost; their potential as irritants and their environmental impact, and for these reasons, the levels of chelating agents has been kept to a minimum.

GB1169551 (Unilever, 1970) describes super-fatted detergent bars containing as preservative a synergistic mixture of at least 0.005 wt% ethane-1- hydroxy-1,1-diphosphonic acid (EHDP) and 0.005 wt% ethylene diamine tetra acetic acid (EDTA) by weight of the bar. The EHDP, the EDTA or both may be in the form of their esters or salts. It is said that by incorporating the above mixture of sequestering agents in a detergent bar, a bar is obtained which is especially resistant to the development of rancidity, spotting or overall discoloration. There is no reference to the antibacterial/antimicrobial efficacy of the resultant bars.

It may be seen from the above-mentioned related art that addition of antibacterial agents to boost the antibacterial/antimicrobial activity, has been the method of choice.

There still is an unmet need for antibacterial personal wash compositions having relatively lower TFM and relatively lower antibacterial agents.

It is an object of the present invention to overcome or ameliorate at least one of the disadvantages of the prior art.

A further object of the present invention is to provide personal wash compositions having relatively lower TFM, which exhibit relatively high antibacterial activity.

It is another object of the invention to provide a personal wash composition which is particularly effective against gram negative bacteria.

It is another object of the present invention to provide a personal wash composition which not only exhibits significant bacteriostatic action (i.e. inhibition of bacterial growth), but also significant bactericidal action (i.e. the ability to kill bacteria)

Other objects of the present invention will become apparent to those skilled in the art by reference to the specification.

The present inventors have surprisingly found that personal wash compositions comprising TFM in the range of 40% to 55% and 0.1 wt% to 1.2 wt% chelating agent exhibit relatively high degree of bacteriostatic and bactericidal activity.

According to the first aspect, the present invention provides a personal wash composition according to claim 1.

Preferably the TFM is from 40% to 50%.

According to the second aspect, the present invention provides use of a personal wash composition according to the first aspect for antibacterial benefit.

According to the third aspect, the present invention provides a method of cleansing skin comprising the step of contacting the skin with a personal wash composition according to the first aspect.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material ought to be understood as modified by the word "about".

It should be noted that in specifying any range of concentration or amount, any particular upper concentration can be associated with any particular lower concentration or amount.

For a more complete understanding of the above and other features and advantages of the invention, reference should be made to the following detailed description of preferred embodiments.

The term Total Fatty Matter, abbreviated to "TFM", is used to denote the percentage by weight of fatty acid and triglyceride residues present in the personal wash composition without taking into account the accompanying cations. For a soap having 18 carbon atoms, an accompanying sodium cation will generally amount to about 8% by weight. Other cations may be employed as desired, for example zinc, potassium, magnesium, alkyl ammonium and aluminium. To calculate the "soap" level in the personal wash composition, the TFM level is to be multiplied by 1.08.

Personal wash compositions may be in a solid form or a non-solid form. Personal wash compositions in solid form are particularly preferred. The total fatty matter is 40% to 55% by weight of the personal wash composition. When the personal wash composition is in solid form, the total fatty matter is preferably 40% to 50%.

It has been observed by the present inventors that lowering of antimicrobial performance as assessed by Minimum Inhibitory Concentration (MIC) and the Minimum Bactericidal Concentration (MBC) test is significant when the total fatty matter (TFM) content in personal wash composition is reduced from 50% to 30%, as against reducing TFM content from 70% to 50%. The predominant wash-active agent in the detergent composition of the present invention is conventional soap, otherwise referred to as fatty acid salt. The soap may be derived from one or a mixture of C₈-C₂₂, preferably C₁₂-C₁₈ straight or branched chain, saturated or unsaturated monocarboxylic acids of natural or synthetic origin. Natural sources e.g. animal, marine or vegetable fats and oils, almost always yield mixtures of these fatty acids, all of which may be employed. Examples thereof include the fatty acids derived from coconut oil, olive oil, palm kernel oil, tall oil, soy bean oil, cottonseed oil, peanut oil, safflower oil, sunflower seed oil, corn oil, fish oils and tallow. Illustratively, individual fatty acids include capric, lauric, myristic, stearic, oleic, palmitic, palmitoleic, ricinoleic, linoleic, and linolenic acid. The fatty acids may also be derived synthetically by paraffin oxidation and oxo-synthesis. The cation or salt portion of the soap is preferably an alkali metal, such as potassium and, especially sodium, but may alternatively be an alkaline earth metal.

A preferred soap is a mixture of about 30% to about 40% coconut oil and about 60% to about 70% tallow. Mixtures may also contain higher amounts of tallow, for example, 0.01% to 20% coconut and remaining tallow. The soaps may contain unsaturation in accordance with commercially acceptable standards. Excessive unsaturation is preferably avoided.

It is essential that the personal wash compositions of the present invention include 0.1 wt% to 1.2 wt% chelating agent as described in claim 1.

The salt form is preferred over acid form, as addition of the chelating agents in the acid form would bring about a corresponding decrease in pH, which is not desirable. It is preferred that the salt of Ethylene Diamine Tetra Acetic acid or Diethylene Triamine Penta Acetic acid is present from 0.1 wt% to 0.4 wt%. It is further preferred that the composition includes a combination of 0.1 wt% to 0.4 wt% salt of Ethylene Diamine Tetra Acetic acid and 0.1 wt% to 0.4 wt% salt of Diethylene Triamine Penta Acetic acid. At significantly low TFM (i.e.TFM from 25% to 55%), the present inventors have observed that salt of EDTA exhibits two optima, at respectively 0.1 wt% and 0.4 wt% for significantly superior performance in terms of bacteriostatic and bactericidal activity, whereas increasing salt of DTPA was observed to continuously increase the antibacterial performance. Of the two, salt of DTPA shows better antibacterial performance. The salt of DTPA exhibited optima at 0.3 wt% and 0.4 wt% in the personal wash composition. With 50% TFM in soap, salt of DTPA showed better efficacy at 0.2 wt% level than salt of EDTA. However with 30% TFM in the composition; salt of DTPA has been found to be better than salt of EDTA even at 0.1 wt% level.

The technical benefit of this invention is in improving the antibacterial benefits while lowering the TFM content of the personal wash composition. It has been observed by the present inventors that by increasing chelating agents in the personal wash composition, especially in bar form, the antibacterial performance against *E*. *coli,* a typical gram negative bacterium, is significantly improved. Transient gram negative bacteria are the cause of diseases and hence improving performance of the personal wash composition against them, by increasing chelating agent level in the personal wash compositions offers significant benefits to the consumers.

Gram-negative bacteria are those bacteria, which do not retain crystal violet dye in the Gram staining protocol. Gram-positive bacteria, on the other hand retain the crystal violet dye when washed in a decolorizing solution. In a Gram stain test, a counter-stain (commonly Safranin) is added after the crystal violet, colouring all Gram-negative bacteria red or pink. The test itself is useful in classifying two distinct types of bacteria based on structural differences in their cell walls. Of these two classes of organisms, Gram negative bacteria are the dominant cause of serious public health diseases such as diarrhoea. It is well known in the prior art that the incidence of diseases such as diarrhoea may be reduced by effective skin cleansing using soap based products.

It has been observed by the present inventors that when acidic filler such as starch is added to the personal wash composition, it reduces the pH of the composition. It has been further observed by the present inventors that the preferred pH of the composition is from 9 to 11.5, and more preferably from 9.8 to 10.8. This can be achieved by addition of an alkali such as sodium hydroxide or sodium carbonate, more preferably sodium carbonate. The preferred level of alkali is from 0.1 wt% to 1.0 wt%, more preferred being 0.25 wt% to 0.5 wt%. The pH of the composition is measured at 30 °C at a concentration of 10 wt%.

In addition to the ingredients described above, the compositions of the present invention preferably include a filler so as to fill the formulation space, arising out of the lower TFM of the composition. A highly preferred filler is starch. Suitable starch materials include natural starch (from corn, wheat, rice, potato, tapioca and the like), pre-gelatinzed starch, various physically and chemically modified starch and mixtures thereof. By the term natural starch is meant starch which has not been subjected to chemical or physical modification; also known as raw or native starch.

A preferred starch is natural or native starch from maize (corn), cassava, wheat, potato, rice and other natural sources of it. Raw starch with different ratio of amylose and amylopectin: e.g. maize (25% amylose); waxy maize (0%); high amylose maize (70%); potato (23%); rice (16%); sago (27%); cassava (18%); wheat (30%) and others. The raw starch can be used directly or can be modified during the process of making the bar composition such that the starch becomes gelatinized, either partially or fully gelatinized.

Another suitable starch is pre-gelatinized which is starch that has been gelatinized before it is added as an ingredient in the present bar compositions. Various forms are available that will gel at different temperatures, e.g., cold water dispersible starch. One suitable commercial pre-gelatinized starch is supplied by National Starch Co. (Brazil) under the trade name FARMAL™ CS 3400 but other commercially available materials having similar characteristics are suitable.

The amount of starch can preferably range from about 5 wt% to 25 wt%, more preferably 6 wt% to 25 wt%, further more preferably 10 wt% to 25 wt%, and most preferably 10 wt% to 15 wt% of the composition.

When it was attempted by the present inventors to fill-in the formulation space with a filler, e.g. starch (due to the lower TFM), it was observed that the pH of the composition dropped significantly, and so did the antibacterial activity. Merely increasing the pH of the composition did not produce any appreciable results.

In addition to TFM and chelating agent, the composition preferably also includes an anionic surfactant at 0.05 wt% to 10 wt%, more preferably 0.5 wt% to 8 wt%, and most preferably 1 wt% to 5 wt%. It has been observed by the present inventors that addition of synthetic surfactant e.g. sodium lauryl sulphate (SLS) boosts the antimicrobial benefit. Especially suitable surfactants are water soluble salts of organic sulphuric reaction products having in the molecular structure an alkyl radical containing from 8 to 22 carbon atoms, and a radical chosen from sulphonic acid or sulphur acid ester radicals and mixtures thereof. Preferred anionic surfactants include sodium lauryl sulphate, alpha olefin sulphonates, sodium lauryl ether sulphate and primary alcohol sulphates.

The other non-soap surfactants such as nonionic, cationic, and zwitterionic, or amphoteric surfactants can also be used for added benefits e.g. for mildness or to boost lather.

Suitable nonionic surfactants can be broadly described as compounds produced by the condensation of alkylene oxide groups, which are hydrophilic in nature, with an organic hydrophobic compound which may be aliphatic or alkyl aromatic in nature. The length of the hydrophilic or polyoxyalkylene radical which is condensed with any particular hydrophobic group can be readily adjusted to yield a water-soluble compound having the desired degree of balance between hydrophilic and hydrophobic elements.

Suitable amphoteric surfactants that optionally can be employed are derivatives of aliphatic secondary and tertiary amines containing an alkyl group of 8 to 18 carbon atoms and an aliphatic radical substituted by an anionic water-solubilizing group, for instance sodium 3-dodecylamino-propionate, sodium 3-dodecylaminopropane sulphonate and sodium N-2-hydroxydodecyl-N-methyltaurate.

Suitable cationic surfactants are quaternary ammonium salts having an aliphatic radical of from 8 to 18 carbon atoms, for instance cetyl trimethyl ammonium bromide.

Suitable zwitterionic surfactants that can be employed are derivatives of aliphatic quaternary ammonium, sulphonium and phosphonium compounds having an aliphatic radical of from 8 to 18 carbon atoms and an aliphatic radical substituted by an anionic water-solubilising group, for instance 3-(N-N-dimethyl-N-hexadecylammonium), propane-1-sulphonate betaine, 3-(dodecylmethyl sulphonium) propane-1-sulphonate betaine and 3-(cetylmethylphosphonium) ethane sulphonate betaine.

Further examples of suitable surfactants are compounds commonly used as surface-active agents given in the well-known textbooks "Surface Active Agents", Volume I by Schwartz and Perry and "Surface Active Agents and Detergents", Volume II by Schwartz, Perry and Berch. The surfactants under this invention are compounds other than soap whose detersive properties, like soap, are due to the presence of a hydrophilic and a hydrophobic group in the molecule.

In addition to the above essential ingredients, the personal wash compositions of the present invention may also comprise any or all of the following ingredients used to increase the shelf life, aesthetics or functionality, namely:
- Vitamins such as vitamin A and E, and vitamin alkyl esters such as vitamin C alkyl esters; lipids such as cholesterol, cholesterol esters, lanolin, ceramides, sucrose esters, and pseudo-ceramides; liposome forming materials such as phospholipids, and suitable amphiphilic molecules having two long hydrocarbon chains; essential fatty acids, poly unsaturated fatty acids, and sources of these materials; triglycerides of unsaturated fatty acids such as sunflower oil, primrose oil, avocado oil, almond oil; vegetable butters formed from mixtures of saturated and unsaturated fatty acids such as shea butter; mineral such as sources of zinc, magnesium, and iron; skin conditioners such as silicone oils, gums and modifications thereof such as linear and cyclic polydimethylsiloxanes, amino, alkyl, and alkylaryl silicone oils; hydrocarbons such as liquid paraffins, petrolatum, Vaseline™, microcrystalline wax, ceresin, squalene, pristan, paraffin wax and mineral oil; conditioning proteins such as milk proteins, silk proteins and glutins; cationic polymers as conditioners which may be used include QUATRISOFT™ LM-200 POLYQUATERNIUM™-24, MERQUAT™ Plus 3330, POLYQUATERMIUM™ 39; and JAGUAR™ type conditioners, humectants such as glycerol, sorbitol, and urea; and emollients such as esters of long chain fatty acids, such as isopropyl palmitate and cetyl lactate.

Further, the composition can be made multicoloured, e.g., striped, through the judicious use of dye as is well known in the art.

Finally, personal wash compositions, when in solid form, generally comprise about 1 wt% to 30 wt%, preferably 2 wt% to 25 wt%, more preferably 3 wt% to 16 wt% water. The amount of water may be significantly higher, up to 90% when the personal wash compositions are in non-solid form.

It is highly preferred that the personal wash compositions of the present invention are in "bar" form.

The soap bars of the invention are preferably prepared by milling and plodding, the melt cast process may also be used. The process may be carried out in any mixer conventionally used in soap manufacture, and is preferably carried out in a high shear-kneading mixer. The preferred mixers include ploughshare mixer, mixers with kneading members of Sigma type, multi-wiping overlap, single curve or double arm. The double arm kneading mixers can be of overlapping or the tangential design. Alternatively, the invention can be carried out in a helical screw agitator vessel, or multi-head dosing pump/high shear mixer and spray drier combinations, as in conventional process.

The invention will now be described in greater detail with reference to the following non-limiting examples.

### EXAMPLES

### Formulations of the personal wash compositions

Various personal wash compositions (in soap-bar form) were made and tested for their antibacterial efficacy. The basic formulations of the compositions used in the examples which follow, are given in Table 1 below:

**Table 1**

| **Ingredients/wt%** | **Compositions** | | | | |
|---|---|---|---|---|---|
| | 70% TFM (A) | 64% TFM (B) | 54% TFM (C) | 48% TFM (D) | 30% TFM (E) |
| Sodium soap of fatty acid * | 75.6 | 69.1 | 58.3 | 51.8 | 32.4 |
| Corn starch | 0 | 0 | 14 | 14 | 25 |
| Talc | 8 | 6 | 5 | 5 | 5 |
| Sorbitol (100%) | 1 | 6 | 6 | 6 | 10 |
| Sodium lauryl sulphate (SLS) | 0 | 2.5 | 4 | 4 | 4.0 |
| Alpha olefin sulfonate (AOS) | 0 | 0 | 0 | 0 | 0 |
| Trichlorocarbanilide | 0.2 | 0.1 | 0.1 | 0.1 | 0.1 |
| Fragrance, color, preservatives and other minors | 2 | 2 | 2 | 2 | 2 |
| Water | to 100% | to 100% | to 100% | to 100% | to 100% |

| | | | | | |
|---|---|---|---|---|---|
| *This number is obtained by multiplying the % TFM by a factor of 1.08. | | | | | |

In experimental bars, wherever a salt of EDTA or salt of DTPA, and/or sodium carbonate was incorporated in the compositions; the same has been indicated in the relevant example; and an equivalent amount of water was reduced from the composition, to keep the sum total of all ingredients in the composition at 100%. The bars were prepared using conventional equipment used in the manufacture of extruded soap. In summary, the bar was prepared by combining soap noodles with the remaining ingredients in Table 1 in a Z-blade mixer and passing the mixture through a 3-roll mill and a refiner. The soap noodles were composed of a mixture of lauric-rich and stearic-rich soaps used in a weight ratio in the range from 40/60 to 10/90. The lauric-rich soaps were derived from palm kernel oil, coconut oil and/or babasu oil. The stearic-rich soaps were derived from tallow, palm oil, palm stearine, hardened soybean oil and crude soybean oil. The compositions so processed were added to the hopper of a two stage extruder and extruded at a temperature of 35°C at an extrusion rate of 1.2 - 4.0 kg/minute through an eyeplate having a 3.5 x 3.5 cm cross section to form billets cut to about 12 cm in lengths. The billets were then transferred to a manual soap stamper and stamped to form the finished personal wash composition in bar form utilizing a die set defining a mould having a volume of approximately 79 to 80 cm³ (density in the range from 1.12 to 1.14 g/cm³).

### Methodology

The following two analytical methods were used to determine the bacterial kill and bacterial growth inhibition of various compositions.

### Bacterial kill assay

All the experiments on this assay were conducted against *E.coli* and as such represent a model of performance against Gram-negative pathogens responsible for diseases such as diarrhoea.

An aqueous (10 wt %) slurry of the personal wash composition (in bar form) is prepared. At time zero, the bacterial suspension is added to this slurry. Addition of this bacterial suspension decreases the product concentration from 10% to 8% (which is a typical in use concentration for soap bars). The test slurries are stirred and after a specified contact time (typically between 30 seconds and 5 minutes) a sample is recovered and transferred to a quenching solution to stop any further antimicrobial activity. The sample is then diluted and placed onto a suitable growth media, such as Tryptone Soy Broth (bacterial growth media) and incubated at a suitable temperature (typically between 28°C and 37 °C). The number of colonies is counted after 18-24 hours of incubation. From this number, the number of bacteria killed whilst in contact with the test slurry is calculated and expressed in terms of log reduction in bacterial numbers. Higher log reduction indicates higher antimicrobial activity in terms of bacterial kill.

### Bacterial growth inhibition assay

An aqueous (10 wt %) slurry of the personal wash composition (in bar form) is prepared. The test slurry is repeatedly diluted in bacterial growth media such as Tryptone Soy Broth. A standard bacterial inoculum of *E. coli* is then added to each dilution of test slurry and the samples are incubated as above. In this way, a series of samples is generated, with varying concentration of the formulation. An effective composition would show appreciable inhibition of growth of bacteria at a comparatively lower concentration than the reference material. The percentage of replicates displaying growth at each dilution was counted to provide an indication of how dilute the product may be while still delivering an antibacterial effect in terms of inhibition of bacterial growth. The more potently antibacterial a product is, the more dilute it may be whilst still inhibiting bacterial growth.

### Example 1

### Effect of TFM on bacterial kill and inhibition of bacterial growth

In this experiment, the effect of varying TFM on bacterial kill and inhibition in the growth of bacteria *(E.coli)* was studied. 10% slurries of each of the Compositions A, D and E (as in Table 1 above) were used for this experiment and the bacterial kill and inhibition was determined by the respective methods given above. All the compositions were devoid of any chelating agent.

**Table 2**

| | % TFM | | |
|---|---|---|---|
| | 30 | 48 | 70 |
| | (Composition E) | (Composition D) | (Composition A) |
| Log reduction | 1.25 | 2.5 | 3.7 |
| % samples displaying growth | 90 | 62 | 45 |

Table 2 shows the effect of TFM on bacterial kill *(E.coli)* at a contact time of 5 minutes, in terms of log reduction in the number of bacteria. Table 2 also shows the percentage of samples displaying growth. Higher growth indicates lesser efficacy.

The results indicate that the ability of the personal wash composition to kill and to inhibit growth of *E.coli* is directly proportional to the TFM.

### Example 2

### Effect of chelating agent on bacterial kill

In this example, the bacterial kill provide by personal wash compositions according to the invention (at 54% and 48% TFM, compositions C and D of Table 1 respectively, with 0.15 wt% pentasodium DTPA) was compared against a commercial 70% TFM soap (composition A of Table 1 devoid of chelating agent) and a comparative 64% TFM soap containing antibacterial active (composition B of Table1 devoid of chelating agent). The kill was determined at a contact time of 2 minutes and 5 minutes for all the compositions by the methodology described above.

**Table 3**

| % TFM | 48% | | 54% | | 64% | | 70% | |
|---|---|---|---|---|---|---|---|---|
| | Composition- D of table-1 with 0.15 wt% Pentasodium salt of DTPA | | Composition- C of table-1 with 0.15 wt% Pentasodium salt of DTPA | | Composition - B of table-1 No chelating agent | | Composition-A of table-1 No chelating agent | |
| | minutes | | minutes | | minutes | | minutes | |
| | 2 | 5 | 2 | 5 | 2 | 5 | 2 | 5 |
| Log reduction | 4.0 | 5.3 | 4.3 | 6.5 | 5.1 | 8.2 | 3.9 | 6.9 |

The results in the above table indicate appreciable log reduction in bacterial numbers (which is a marker of bacterial kill) at relatively lower TFM of 48% and 54%, in the presence of 0.15 wt% pentasodium DTPA.

### Example 3

### Effect of chelating agent on inhibition of bacterial growth

In this experiment, the inhibition of bacterial growth of 48% and 30% TFM compositions containing varying levels of disodium EDTA and pentasodium DTPA (compositions C and D of Table 1 respectively, containing varying levels of chelating agents) was compared against a commercial 70% TFM soap (composition A of table-1, devoid of chelating agent). The bacterial inhibition was determined by the method given above. Results of this experiment are presented in Table 4 below.

**Table 4**

| % TFM | % chelating agent | Percentage of samples displaying growth |
|---|---|---|
| 48% | 0.1 | 40 |
| (Composition D of Table 1 containing disodium salt of EDTA) | 0.2 | 45 |
| | 0.3 | 40 |
| | 0.4 | 42 |
| | 0.5 | 40 |
| 48% | 0.1 | 39 |
| (Composition D of Table 1 containing Pentasodium salt of DTPA) | 0.2 | 42 |
| | 0.3 | 27 |
| | 0.4 | 15 |
| | 0.5 | 8 |
| 30% | 0.3 | 15 |
| (Composition E of Table1 containing pentasodium salt of DTPA) | 0.4 | 17 |
| 70% | 0 | 47 |
| Composition A of Table 1 | | |

The results in Table 4 indicate that the addition of pentasodium DTPA or disodium EDTA to a 48% TFM soap composition leads to relatively higher inhibition of bacterial growth, when compared to a 70% TFM commercial soap devoid of chelating agent. Similar results can be seen upon inclusion of pentasodium DTPA in 30% TFM soap.

### Example 4

### Effect of chelating agent and pH on bacterial kill

In this experiment, the effect of chelating agent and pH on bacterial kill was studied on a 48% TFM soap bar (composition D of Table 1) containing 0.15 wt% pentasodium salt of DTPA and varying levels of Na₂CO₃, to vary the pH of the compositions. An equivalent amount of water was reduced from the compositions to compensate for the added ingredients. The kill was determined at a contact time of 2 minutes and 5 minutes for all the compositions, by the methodology described above. The data on bacterial kill is presented in Table 5 below. The Table also includes comparative results obtained with commercial 70% TFM bars devoid of chelating agent (composition A of Table 1 devoid of chelating agent), as well as a 64% TFM soap devoid of chelating agent (composition B of Table 1 devoid of chelating agent). The pH of the compositions was measured at 10% aqueous solution concentration at 30°C.

**Table 5**

| **% TFM** | **wt% chelating agent, and wt% Na₂CO₃** | **pH** | **Log reduction** | |
|---|---|---|---|---|
| | | | 2 minutes | 5 minutes |
| 48 | 0.15; 0.2 | 10.2 | 5.4 | 8.2 |
| 48 | 0.15; 0.5 | 10.8 | 6.2 | 7.0 |
| 48 | 0.15; 1.0 | 11.5 | 7.5 | 8.0 |
| 64 | 0; 0 | 10.62 | 5.0 | 8.2 |
| 70 | 0; 0 | 10.45 | 4.0 | 7.0 |

The data indicates that increasing pH further improved the antibacterial effect of the chelating agent at 48% and 54% TFM soap compositions with chelating agents.

### Example 5

### Effect of varying levels of chelating agent and pH on bacterial kill

In another set of experiments, the effect of varying levels of chelating agent and pH, while keeping the TFM constant was studied. In these experiments, a series of 48% TFM soap compositions (composition D of Table 1) were prepared, which contained varying levels of chelating agent (pentasodium DTPA) and sodium carbonate (to vary the pH). An equivalent amount of water was reduced from the compositions to compensate for the added ingredients. These compositions were tested for bacterial kill in a 2 minute contact time experiment by the method described above. The results of these experiments are presented in Table 6 below. For comparison, the results were compared against 70% TFM and 64% TFM soap compositions, devoid of chelating agent or sodium carbonate (compositions A and B of Table 1 respectively).

**Table 6**

| **% TFM** | **Pentasodium DTPA /%wt** | **Na₂CO₃ level/wt%** | **pH** | **Log reduction** |
|---|---|---|---|---|
| 48% | 0.1 | 0.1 | 10.44 | 5.2 |
| | 0.15 | 0.1 | 10.45 | 7.1 |
| | 0.2 | 0.1 | 10.39 | 7.1 |
| | 0.1 | 0.2 | 10.65 | 7.1 |
| | 0.15 | 0.2 | 10.65 | 7.1 |
| | 0.2 | 0.2 | 10.64 | 7.1 |
| | 0.1 | 0.25 | 10.77 | 7.0 |
| | 0.15 | 0.25 | 10.82 | 7.0 |
| | 0.2 | 0.25 | 10.83 | 7.0 |
| | 0.1 | 0.5 | 10.75 | 7.0 |
| | 0.15 | 0.5 | 10.81 | 7.0 |
| | 0.2 | 0.5 | 10.83 | 7.0 |
| | 0.1 | 1.0 | 10.91 | 7.0 |
| | 0.15 | 1.0 | 10.88 | 7.0 |
| | 0.2 | 1.0 | 10.92 | 7.0 |
| 70% | - | - | 10.45 | 5.8 |
| 64% | - | - | 10.62 | 6.4 |

The above results indicate that a combination of pH and chelating agent gives comparatively higher log reduction, even at a short contact time of 2-minutes.

### Example 6

### Effect of combination of chelating agents

In yet another set of experiments, the effect of a combination of chelating agents on the bacterial kill (log reduction at 2 minute contact time) was studied for the compositions having the formulation as given in Table 7 below.

**Table 7**

| Ingredients (%) | Composition | | |
|---|---|---|---|
| | F | G | H |
| Sodium Soap | 52 | 52 | 52 |
| Corn Starch | 14.00 | 14.00 | 14.00 |
| Talc | 5.00 | 5.00 | 5.00 |
| Sorbitol (100%) | 6.00 | 6.00 | 6.00 |
| Titanium Dioxide | 0.40 | 0.40 | 0.40 |
| Disodium EDTA | 0.04 | 0.04 | 0.04 |
| EHDP | 0.02 | 0.02 | 0.02 |
| Pentasodium DTPA | 0.20 | 0.20 | 0.10 |
| Sodium Carbonate | 0.00 | 0.25 | 0.10 |
| Sodium Lauryl Sulphate | 2.00 | 2.00 | 2.00 |
| Alpha Olefin Sulphonate | 2.00 | 2.00 | 2.00 |
| Moisture | 16.00 | 16.00 | 16.00 |
| Fragrance, color and other minors | To 100 | To 100 | To 100 |

The TFM of all compositions was 48%, and the soap bars were prepared by the method used to prepare soap bars of compositions in Table 1. The bacterial kill efficacy of above compositions was tested as per methodology given above, and the results obtained are presented in Table 8 below. The results have been compared with that of 64% TFM and 70% TFM soap compositions of Table 1.

**Table 8**

| Composition | Log reduction | pH |
|---|---|---|
| A (of Table 1) | 4.9 | 10.45 |
| B (of Table 1) | 6.9 | 10.62 |
| F | 6.5 | 10.57 |
| G | 7.0 | 10.65 |
| H | 6.2 | 10.62 |

Thus, it can be readily seen that combinations of chelating agents also provide comparatively higher log reduction.

It will be appreciated that the illustrated examples, provide for personal wash compositions having relatively low TFM having relatively high degree of antibacterial activity. It will also be appreciated that the illustrated examples provide personal wash compositions which are particularly effective against Gram negative bacteria *E.coli.* It will also be appreciated that the illustrated examples provide personal wash compositions which not only exhibit significant bacteriostatic action (i.e. inhibition of bacterial growth), but also significant bactericidal action (i.e. the ability to kill bacteria)

It should be understood that the specific forms of the invention herein illustrated and described are intended to be representative only as certain changes may be made therein without departing from the clear teachings of the disclosure.

Although the invention has been described with reference to specific embodiments, it will be appreciated by those skilled in the art that the invention may be embodied in many other forms.

## Claims

1. A personal wash composition comprising:
(i) 40% to 55% Total Fatty Matter; and
(ii) 0.1 wt% to 1.2 wt% chelating agent comprising a salt of Diethylene Triamine Penta Acetic acid;
wherein the composition is in the bar form and the term TFM denotes the percentage by weight of fatty acid and triglyceride residues present in the personal wash composition without taking into account the accompanying cations.

2. A personal wash composition as claimed In claim 1 comprising 3-25% by weight water.

3. A personal wash composition as claimed in claim 1 or claim 2 wherein said salt of Diethylene Triamine Penta Acetic acid Is from 0.1 wt% to 0.4 wt%.

4. A personal wash composition as claimed in any one of the preceding claims wherein said salt of Diethylene Triamine Penta Acetic acid is a Pentasodium Diethylene Triamine Penta Acetic acid.

5. A personal wash composition as claimed in any one of the preceding claims comprising 5 wt% to 25 wt% starch.

6. A personal wash composition as claimed in any one of the preceding claims wherein the Total Fatty Matter is 40% to 50% when the personal wash composition is in solid form.

7. A personal wash composition as claimed in any one of the preceding claims wherein pH of the composition is from 9 to 11.5.

8. A personal wash composition as claimed in claim 7 wherein the pH of the composition is from 9.8 to 10.8.

9. A personal wash composition as claimed in any one of the preceding claims comprising 0.05 wt% to 10 wt% anionic surfactant.

10. A personal wash composition as claimed in claim 9 wherein said anionic surfactant is selected from Sodium lauryl sulphate, alpha Olefin Sulphonates, Sodium Lauryl Ether Sulphate or Primary alcohol sulphates.

11. A cosmetic non-therapeutic method of cleansing skin comprising the step of contacting the skin with a personal wash composition according to claim 1.

## Patentansprüche

1. Zusammensetzung für die Körperwäsche,
die Folgendes aufweist:
(i) 40 bis 55 % gesamtes Fettmaterial und
(ii) 0,1 bis 1,2 Gew.-% Komplexbildner, der ein Salz von Diethylentriaminpentaessigsäure aufweist;
wobei die Zusammensetzung in Form eines Stücks vorliegt und der Begriff TFM für den Gewichtsprozentsatz von Fettsäure- und Triglyceridresten steht, der in der Zusammensetzung für die Körperwäsche vorliegt, ohne dass begleitende Kationen in Betracht gezogen sind.

2. Zusammensetzung für die Körperwäsche nach Anspruch 1, die 3 bis 25 Gew.-% Wasser aufweist.

3. Zusammensetzung für die Körperwäsche nach Anspruch 1 oder 2, wobei das Salz von Diethylentriaminpentaessigsäure 0,1 bis 0,4 Gew.-% ausmacht.

4. Zusammensetzung für die Körperwäsche nach einem der vorstehenden Ansprüche, wobei das Salz von Diethylentriaminpentaessigsäure Pentanatriumdiethylentriaminpentaessgisäure ist.

5. Zusammensetzung für die Körperwäsche nach einem der vorstehenden Ansprüche, die 5 bis 25 Gew.-% Stärke aufweist.

6. Zusammensetzung für die Körperwäsche nach einem der vorstehenden Ansprüche, wobei das gesamte Fettmaterial 40 bis 50 % ausmacht, wenn die Zusammensetzung für die Körperwäsche in einer festen Form vorliegt.

7. Zusammensetzung für die Körperwäsche nach einem der vorstehenden Ansprüche, wobei der pH-Wert der Zusammensetzung 9 bis 11,5 beträgt.

8. Zusammensetzung für die Körperwäsche nach Anspruch 7, wobei der pH-Wert der Zusammensetzung 9,8 bis 10,8 beträgt.

9. Zusammensetzung für die Körperwäsche nach einem der vorstehenden Ansprüche, die 0,05 bis 10 Gew.-% anionisches Tensid aufweist.

10. Zusammensetzung für die Körperwäsche nach Anspruch 9, wobei das anionische Tensid aus Natriumlaurylsulfat, α-Olefinsulfonaten, Natriumlaurylethersulfat oder primären Alkoholsulfaten ausgewählt ist.

11. Kosmetisches, nicht-therapeutisches Verfahren zum Reinigen der Haut, das den Schritt des Inkontaktbringens der Haut mit einer Zusammensetzung für die Körperwäsche nach Anspruch 1 aufweist.

## Revendications

1. Composition de lavage pour l'hygiène personnelle comprenant :
(i) de 40 % à 55 % de matière grasse totale ; et
(ii) de 0,1 % en poids à 1,2 % en poids d'agent chelatant comprenant un sel d'acide diéthylènetriaminepentaacétique ;
dans laquelle la composition est dans la forme d'un pain et le terme TFM indique le pourcentage en poids de résidus d'acide gras et de triglycéride présents dans la composition de lavage pour l'hygiène personnelle sans tenir compte des cations accompagnant.

2. Composition de lavage pour l'hygiène personnelle selon la revendication 1 comprenant 3-25 % en poids d'eau.

3. Composition de lavage pour l'hygiène personnelle selon la revendication 1 ou la revendication 2 dans laquelle ledit sel d'acide diéthylènetriaminepentaacétique est présent pour de 0,1 % en poids à 0,4 % en poids.

4. Composition de lavage pour l'hygiène personnelle selon l'une quelconque des revendications précédentes dans laquelle ledit sel d'acide diéthylènetriaminepentaacétique est un acide pentasodium diéthylènetriaminepentaacétique.

5. Composition de lavage pour l'hygiène personnelle selon l'une quelconque des revendications précédentes comprenant de 5 % en poids à 25 % en poids d'amidon.

6. Composition de lavage pour l'hygiène personnelle selon l'une quelconque des revendications précédentes dans laquelle la matière grasse totale est de 40 % à 50 % lorsque la composition de lavage pour l'hygiène personnelle est dans une forme solide.

7. Composition de lavage pour l'hygiène personnelle selon l'une quelconque des revendications précédentes dans laquelle le pH de la composition est de 9 à 11,5.

8. Composition de lavage pour l'hygiène personnelle selon la revendication 7 dans laquelle le pH de la composition est de 9,8 à 10,8.

9. Composition de lavage pour l'hygiène personnelle selon l'une quelconque des revendications précédentes comprenant de 0,05 % en poids à 10 % en poids de tensioactif anionique.

10. Composition de lavage pour l'hygiène personnelle selon la revendication 9 dans laquelle ledit tensioactif anionique est choisi parmi le laurylsulfate de sodium, des sulfonates d'oléfines alpha, le lauryléthersulfate de sodium et des sulfates d'alcools primaires.

11. Procédé non-thérapeutique cosmétique de nettoyage de la peau comprenant l'étape de mise en contact de la peau avec une composition de lavage pour l'hygiène personnelle selon la revendication 1.
